# EUROPEAN PATENT APPLICATION

(11) **EP 2 671 608 A2**
(43) Date of publication of application: **11.12.2013**
(21) Application number: 13170938.8
(22) Date of filing: 06.06.2013
(51) Int. Cl.: A61M 35/00

(54) **Low profile handle pneumatic assist device for dispensing dual materials**

(30) Priority: 07.06.2012 US 201261657008 P; 13.03.2013 US 201313798268
(71) Applicant: Nordson Corporation, Westlake, OH 44145 (US)
(72) Inventor: Kirk, Thomas Allan, Hastings, MN Minnesota 55033 (US); Sharratt, Todd William, Stillwater, MN Minnesota 55082 (US); Lou, Huadong, 313 Maple Plain, MN Minnesota 55108 (US)
(74) Representative: Eisenführ, Speiser & Partner

(57) **Abstract**

A gas-assisted fluid-dispensing device configured to deliver an aerosol onto a surgical site, and methods of use. The device includes a support structure and a fluid chamber. The fluid chamber contains a fluid and has a distal end and a proximal end. A piston is positioned within the fluid chamber. A spray nozzle tip is fluidly coupled to the distal end of the fluid chamber and includes a gas inlet. The tip generates the aerosol with a gas from the gas inlet and the fluid from the fluid chamber. A pneumatic switch has a first stage and a second stage. The first stage permits gas flow through the pneumatic switch and into the gas inlet of the tip. The second stage permits gas flow through the pneumatic switch and into both the gas inlet of the tip and into the fluid chamber.

## Description

### Cross-Reference to Related Applications

This application claims the priority of U.S. Provisional Patent Application Serial No. 61/657,008, filed on June 7, 2012 (pending), the disclosure of which is incorporated by reference herein in its entirety.

The disclosures of International Patent Application No. PCT/US11/29763, filed on March 24, 2011 and entitled GAS-ASSISTED FLUID DISPENSING DEVICE and U.S. Application Serial No. 61/657,004, filed June 7, 2012 and entitled MECHANICAL ASSIST DEVICE WITH KEYED COMPONENT FEATURES FOR HIGHLY REACTIVE AND MULTI-VISCOSITY BIOMATERIALS are incorporated herein by reference in their entireties.

### Field of the Invention

The present invention relates generally to fluid-dispensing devices and, more particularly, to a fluid-dispensing device configured to dispense aerosols.

### Background of the Invention

In the medical field, a surgeon routinely needs to deliver a drug or another fluid to an anatomical surface within a surgical site in a patient. Conventional manual and non-manual syringes are often used to deliver these fluids to the surgical site. For example, one known conventional syringe design includes two barrels, each containing separate fluids that are simultaneously dispensed and mixed to form a coating adapted to prevent bleeding at the surgical site. In order to spread the coating over a surface area at the surgical site, the double-barreled syringe may be coupled to a known mixing or blending spray tip, such as the FIBRIJET brand of blending tips, such as model SA-3654, that is commercially available from Micromedics of St. Paul, Minnesota. The blending spray tip receives the fluids from each of the two barrels, along with a pressurized gas from a pressurized gas source, to form a therapeutic aerosol that is sprayed over the surface to be coated. The therapeutic aerosol, including, for example, pain relievers, antibiotics, or coagulants, may be applied to the surgical site before, during, or after a surgical procedure.

It is widely accepted that a maximum of about 8 pounds of force should be applied to the syringes during use of such gas-assisted dispensers. However, as utility of the dispensers increases to include viscous materials and/or larger fluid containers (barrels, syringes, and so forth) the maximum force is often exceeded. This not only presents concerns for the structural integrity of the dispenser but also increases the discomfort experienced by those users having smaller hands. Thus, there exists a need for ergonomically improved dispensing devices that more efficiently dispense fluids, particularly high viscosity fluids, while increasing user comfort and reducing hand strain.

### Summary

In accordance one illustrative embodiment, a gas-assisted fluid-dispensing device is provided and delivers an aerosol onto a surgical site. The fluid dispensing device generally includes a support structure which, for example, may be a handle. A fluid chamber is coupled with the support structure and is configured to contain a fluid therein. The fluid chamber includes a distal end and proximal end. A piston is positioned within the fluid chamber. A spray nozzle tip is fluidly coupled to the distal end of the fluid chamber and includes a gas inlet. The spray nozzle tip is capable of generating the aerosol with a gas from the gas inlet and the fluid from the fluid chamber. A pneumatic switch is operably coupled to the support structure and has a first stage configured to permit gas flow through the pneumatic switch and into the gas inlet of the spray nozzle tip and a second stage configured to permit gas flow through the pneumatic switch and into both the gas inlet of the spray nozzle tip and into the fluid chamber for moving the piston and forcing the fluid from the fluid chamber into the spray nozzle tip.

In a more specific embodiment, the fluid dispensing device comprises first and second fluid chambers coupled with the support structure and configured to contain respective first and second fluids therein. Each of the first and second fluid chambers includes a piston and the spray nozzle tip is fluidly coupled to the distal ends of the first and second fluid chambers for generating the aerosol using the gas from the gas inlet and the first and second fluids from the first and second fluid chambers. Actuation of the pneumatic switch into the first stage permits gas flow through the pneumatic switch and into the gas inlet of the spray nozzle tip, while actuation into the second stage permits gas flow through the pneumatic switch and into both the gas inlet of the spray nozzle tip and into the first and second fluid chambers for moving the respective first and second pistons and forcing the fluids from the first and second fluid chambers into the spray nozzle tip. As additional aspects, a holder is provided, and the support structure further comprises a handle. The holder is configured to support the fluid chamber or chambers therein and the holder is operably coupled to the handle. The handle, the holder and the pneumatic switch may be formed as a unitary piece. The spray nozzle tip, the handle and the fluid chamber or chambers may be coplanar.

The holder may be configured to couple first and second fluid chambers to the handle and the holder may include first and second channels configured to receive respective ones of the first and second fluid chambers. First and second keyed adapters are coupled to respective distal ends of the first and second fluid chambers. The first channel includes a projection configured to receive the keyed adaptor of the first fluid chamber. An arm may extend distally away from the holder toward the spray nozzle tip and includes a keyed surface. The spray nozzle tip includes a keyed groove configured to receive the keyed surface of the arm.

In another aspect, a first fluid line is in fluid communication with the pneumatic switch and with a gas inlet that is operably coupled to the support structure. A second fluid line is in fluid communication with the pneumatic switch and with a gas inlet of the spray nozzle tip. A third fluid line is in fluid communication with the pneumatic switch and with the fluid chamber or chambers. In the first stage, the pneumatic switch fluidically couples the first fluid line with the second fluid line for supplying gas to the spray nozzle tip. In the second stage the pneumatic switch fluidically couples the first fluid line with the second and third fluid lines for supplying gas to the spray nozzle tip and also to the fluid chamber or chambers for moving the piston or pistons and forcing the fluid(s) from the fluid chamber(s) into the spray nozzle tip.

In an additional aspect, a fluid dispensing device is provided and includes a fluid chamber having a distal end and a proximal end, and the fluid chamber is configured to contain a fluid. A piston is positioned within the fluid chamber. A plug is inserted into the proximal end of the fluid chamber and the plug includes a plug outlet. A gas inlet is operably coupled to the plug outlet to provide pressurized gas into the fluid chamber. The piston seals the proximal end of the fluid chamber such that the pressurized gas applies pressure to the piston.

In another aspect, a method of using a gas-assisted fluid-dispensing device for delivering an aerosol onto a surgical site is provided. The method generally comprises actuating the pneumatic switch into a first stage to permit gas flow into the gas inlet of the spray nozzle tip, and actuating the pneumatic switch into a second stage to permit gas flow through the pneumatic switch and into both the gas inlet of the spray nozzle tip and into the fluid chamber or chambers for moving the piston or pistons and forcing the fluid(s) from the fluid chamber(s) into the spray nozzle tip.

Various additional features and advantages of the invention will become more apparent to those of ordinary skill in the art upon review of the following detailed description of the illustrative embodiments taken in conjunction with the accompanying drawings.

### Brief Description of the Drawings

FIG. 1 is a perspective view illustrating a handle and holder portion of a gas-assisted fluid-dispensing device constructed in accordance with an illustrative embodiment of the invention.

FIG. 2 is an elevational view of the gas-assisted fluid-dispensing device shown in FIG. 1, and partially fragmented to show internal details of the fluid chambers.

FIGS. 3A-3C are respective cross sectional views of a pneumatic switch associated with the device shown in FIGS. 1 and 2, and illustrating respective positions of the switch.

FIG. 4 is an enlarged perspective view of the distal end portion of the device shown in FIG. 2.

### Detailed Description

Turning now to the figures, a gas-assisted fluid dispenser 10 in accordance with one embodiment of the present invention is shown and described in detail. The dispenser 10 includes a fluid-dispensing device 12 having at least one fluid source. The fluid source may include, for example, a syringe 13 having one or more parallel fluid chambers (two fluid chambers 16, 17 are shown) for simultaneously dispensing one or more fluids 14, 15, such as topical or therapeutic medicinal agents. Moreover, it would be readily appreciated that if more than one fluid 14, 15 is administered, equal volume need not be dispensed. Instead, the volume of a first fluid 14 dispensed from the first fluid chamber 16 may be larger than the volume of a second fluid 15 dispensed from the second fluid chamber 17. Each of the fluid chambers 16, 17 includes a tapered distal end 18, a proximal end 20, and a cylinder 22 extending therebetween. A floating piston 24 is within each cylinder 22. A spray nozzle tip 26 is coupled to the syringe(s) 13. And, because the syringe(s) 13 of the illustrative embodiment includes two fluid chambers 16, 17, the spray nozzle tip 26 is illustrated as a Y-connector attached to both tapered distal ends 18 of the fluid chambers 16, 17. The spray nozzle tip 26 further includes a gas inlet, illustrates as a luer fitting 120, configured to receive a spray gas line 28. Thus, the spray nozzle 26, as shown, includes two fluid inlets, one gas inlet, and one outlet. One of ordinary skill in the art would readily appreciate that the shape of the outlet may be configured to provide a desired aerosol effect. That is, a desired direction and/or spread of the resultant aerosol, along with the gas pressure, may be determined by incorporating a particular design for the outlet.

The first and second fluid chambers 16, 17 are supported within a source holder 30, which may have a molded polymeric material construction that is sized and shaped to accommodate the fluid chambers 16, 17. Accordingly, various sizes, shapes, and configurations of holders are possible for supporting one or more fluid chambers 16, 17 of similar or varying sizes. In the illustrative embodiment, the holder 30 includes first and second channels 32, 33 that are shaped and sized to receive the two fluid chambers 16, 17, respectively.

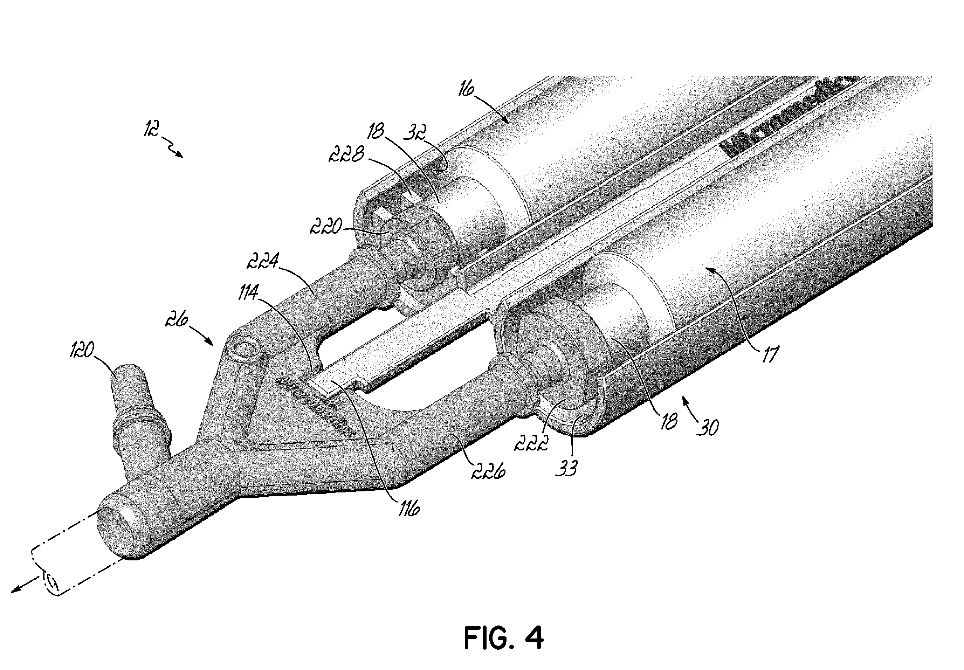

To facilitate consistent assembly of the fluid dispensing device 12, that is a first fluid chamber 16 containing a first fluid 14 is loaded into the first channel 32 while a second fluid chamber 17 containing a second fluid 15 is loaded into the second channel 33, the distal tapered ends 18 of the fluid chambers 16, 17 may include keyed adaptors 220, 222, embodiments of which are shown in FIG. 4. The first and second keyed adaptors 220, 222 may vary in at least one structural dimension, which, as shown, are different diameters. Each adaptor 220, 222 further includes a distally-expending port, such a luer, configured to be coupled to the inlets 224, 226 of the nozzle tip 26.

In addition to the keyed adaptors 220, 222, the distal ends of the first and second channels 32, 33 of the holder 30 may be particularly configured to receive one of the two keyed adaptors 220, 222. More specifically and in accordance with the exemplary embodiment, the first channel 32 includes a structured surface, such a projection, which is illustrated herein as one or more ribs 228 that reduce the inner diameter of the first channel 32 to approximately the outer diameter of the first keyed adaptor 220. Meanwhile, the second channel 33, remaining devoid of diameter-reducing ribs, remains sufficiently large so as to receive the larger outer diameter of the second keyed adaptor 222. As a result, and when the fluid chambers 16, 17 are loaded into the holder 30, the first fluid 14 within the first fluid chamber 16 is necessarily loaded into the first channel 32 of the holder 30 having the ribs 228 therein to support the smaller outer diameter adaptor 220. The second fluid chamber 17 containing the second fluid 15 is then necessarily loaded into the second channel 33.

With the fluid chambers 16, 17 coupled to the holder 30, the nozzle tip 26 may further include an indicia, such as the imprinted donut of the first arm of the Y-connector, which would be understood to reside on the same side during each use of the nozzle tip 26. However, use is not prevented by such indicia and a user that is quickly assembling the dispenser 10 or otherwise unaware of the laboratory or surgical suite's standard operating procedures may inadvertently rotate the nozzle tip 26 such that the indicia is flipped to the opposing side during use. While this switch may be seemingly benign, in those embodiments where the first fluid reacts with the second fluid to form a coagulant or other extremely viscous, gelatinous, or adhesive material, this simple reversal of the Y-connector with respect to the first and second fluids may result in a chemical reaction within the channels of the Y-connector with residual first and second fluids therein. With sufficient residual material and/or reactivity, the channels of the Y-connector may become clogged, rendering the nozzle tip inoperable.

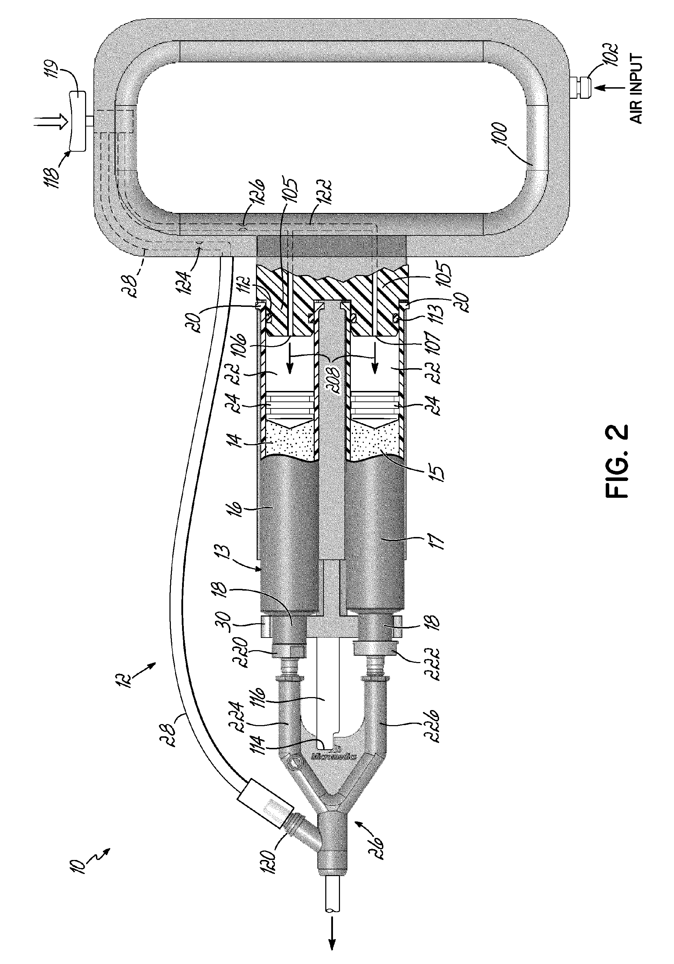

As shown in FIG. 2, to prevent such reversal of the nozzle tip, the Y-connector may be keyed to a groove 114 so as to receive an arm 116 protruding distally from the holder, which prevents reversal of the nozzle tip 26 from its accepted arrangement with the fluid chambers.

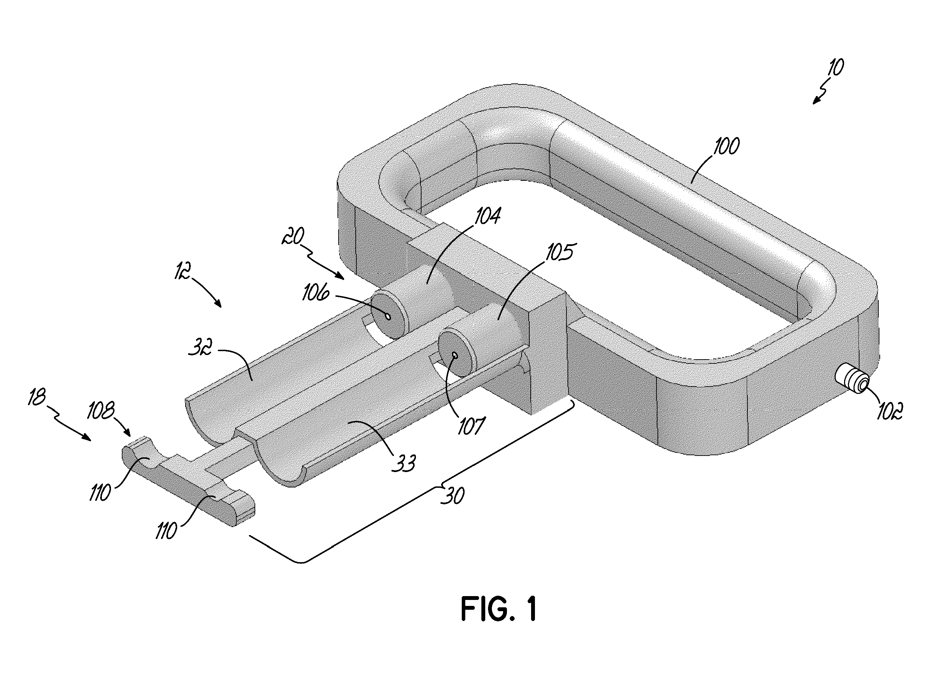

With respect to FIG. 1, the dispenser 10 further includes a handle 100 for conveniently gripping the fluid dispensing device 12. An input fitting 102 operably coupled to the handle 100 is adapted to an air input in order to operably provide pressurized gas to the dispenser 10. According to the illustrative embodiment of the present invention, the handle 100 is positioned between the fluid dispensing device 12 and the input fitting 102. Thus, gas delivered through the air input moves within and through the handle 100 and in fluid communication with the fluid dispensing device 12 via a plurality of gas lines 28, 122 (FIG. 2). However, it will be appreciated that many configurations of the handle 100 relative to the input fitting 102 and the fluid dispensing device 12 are possible to operably deliver pressurized gas to the fluid dispensing device 12.

The fluid dispensing device 12 generally includes the source holder 30 with the channels 32, 33 for operably supporting the fluid chambers 16, 17 (FIG. 2). The source holder 30 may be affixed to the handle 100 or removable affixed to the handle 100. In the case of the removably affixed source holder 30, various sizes of source holders 30 may be interchanged to accommodate syringes 13 of different size. In the alternative, the source holder 30 may be affixed to the handle 100; however, the source holder 30 may be molded of differing sizes to accommodate syringes 13 of different size. In addition, the fluid dispensing device 12 includes plugs 104, 105 configured to operably engage the proximal ends 20 of the fluid chambers 16, 17 and in axial alignment with the respective channels 32, 33. Concentrically located within the plugs 104, 105 are plug outlets 106, 107 in the form of a through hole operably connectable to the air input such that a pressurized gas may travel from the input fitting 102 to the plug outlets 106, 107. Moreover, the plugs 104, 105 are positioned so as to receive the proximal ends 20 of each fluid chamber 16, 17. It will be appreciated that the fluid dispensing device 12 may be adapted for any number of syringes 13 or fluid chambers 16, 17 in the above manner. According to this embodiment of the present invention, the source holder 30 is adapted for two fluid chambers 16, 17. Thus, the fluid dispensing device 12 includes a first plug 104 and first plug outlet 106 in alignment with a first channel 32 for receiving a first fluid chamber 16 and a second plug 105 and second plug outlet 107 in alignment with a second channel 33 for receiving a second fluid chamber. Preferably, the spray nozzle tip 26, the handle 100, and the source holder 30 are co-planar such that the dispenser 10 is configured with a line of sight around this plane from the operator's perspective of use.

A channel extension member 108 may also be attached to the source holder 30 and positioned at the distal ends 18 of the fluid chambers 16, 17 as shown. The channel extension member 108 further includes channel extension grooves 110 keyed to each of the fluid chambers 16, 17. Each channel extension groove 110 further prevents the use of incorrect fluid chambers 16, 17 and improves support and alignment of the proper fluid chambers 16, 17.

As shown in FIG. 2, each of the fluid chambers 16, 17 are inserted onto the respective plugs 104, 105 and configured to form a seal thereagainst. More particularly, each plug 104, 105 includes an O-Ring 112, 113 adapted to seal pressurized air within the cylinders 22 delivered via the plug outlets 106, 107. The adaptors 220, 222 are further attached to the distal end 18 of the fluid chambers 16, 17 and configured to uniquely fit the spray nozzle tip 26. The spray nozzle tip 26 also includes a groove 114 keyed to an arm 116 upon installation onto the adapters 220, 222. The arm 116 is attached to the source holder 30 near the distal end 18 of the fluid chambers 16, 17 and extends between the spray nozzle tip 26 and the source holder 30. As such, the spray nozzle tip 26 may only be installed in one possible orientation, which helps to further reduce the chance of incorrect installation.

According to the present embodiment, pressurized gas is operably delivered to the spray nozzle tip 26 and plug outlets 106, 107 via the air input at the input fitting 102. In addition, a switch 118 is installed between the input fitting 102 and both the spray nozzle tip 26 and plug outlets 106, 107 to operably control delivery of pressurized gas to the spray nozzle tip 26 and the plug outlets 106, 107. The pressurized gas is operably connected to the switch 118 from the input fitting 102. An operator selectively operates the switch 118 to deliver pressurized gas to the spray nozzle tip 26 and/or the plug outlets 106, 107. According to the present embodiment, the switch 118 is a three-way switch such that pressurized gas is selectively delivered to either the spray nozzle tip 26 or both the spray nozzle tip 26 and the plug outlets 106, 107. Preferably, the handle 100, the switch 118, and the source holder 30 are formed as a unitary piece; however, it will be appreciated that any one of these components may be affixed together as separate pieces.

The spray nozzle tip 26 is in fluid communication with the pressurized gas at the switch 118 via the sprayer gas line 28. As shown in FIG. 2, the sprayer gas line 28 has a tube portion and an internal handle portion. The spray nozzle tip 26 further includes the luer fitting 120 to which the tube portion of the sprayer gas line 28 is attached at one end. The other end of the tube portion is attached to the handle 100 at the internal handle portion. However, the plug outlets 106, 107 are in fluid communication with the pressurized gas via a material delivery gas line 122. As shown, the material delivery gas line 122 is located within the handle 100. Each of the gas lines 28, 122 within the handle 100 may be molded within the handle 100; however, it will be appreciated that the pressurized gas may be delivered to either the spray nozzle tip 26 or the plug outlets 106, 107 via any known method.

Moreover, the sprayer gas line 28 and the material delivery gas line 122 include first and second orifices 124, 126 therein respectively. Each of the orifices 124, 126 serves to limit the delivery of the pressurized gas to the spray nozzle tip 26 and the plug outlet 106. While either orifice 124, 126 sizes may vary depending on the amount of pressure delivered by the pressurized gas or the viscosity of the material, the first orifice 124 generally has a larger diameter than the second orifice 126. The pressurized gas may be pressurized carbon dioxide and delivered at a maximum of about 20 psi and 5 liters per minute. In the event the switch 118 is generally fully open, the first orifice 124 may be configured to permit approximately 20 psi of inflow pressure delivered at 5 liters per minute and the second orifice 126 may be configured to permit approximately 5 psi of inflow pressure delivered at 0.5 liters per minute. Once the pressurized gas pressurizes the fluid chambers 16, 17 via the plug outlets 106, 107, fluids 14, 15 are forced into and through the spray nozzle tip 26.

As the fluids 14, 15 are released from the fluid chambers 16, 17 into the spray nozzle tip 26, the dispensing gas, which is also moving through the spray nozzle tip 26, is mixed with and atomizes, or disperses, the fluids 14, 15 as a treatment aerosol from the outlet of the spray nozzle tip 26. The surgeon may continue dispensing the treatment aerosol by further compressing the switch 118. Without further compression of the selectable switch 118, only the dispensing gas is released from the spray nozzle tip 26. On one hand, in order to provide good aerosol from the start and to clear mixed fluids 14, 15 from the lines of the nozzle tip 26, the operator may select only to pressurize the sprayer gas line 28. On the other hand, the operator may fully compress the switch 118 to deliver fluids 14, 15 from the fluid chambers 16, 17 into the spray nozzle tip 26. Before, during, and after each switch squeeze, the dispensing gas is directed into the luer gas fitting 120 of the nozzle tip 26. In this way, the gas flow may be initiated prior to the release fluids from the fluid chambers into the spray nozzle tip 26 such that when the fluids are released, the fluids mixed with the flowing gas, atomizes or disperses, as a treatment aerosol from the outlet of the spray nozzle tip. The surgeon may continue dispensing the treatment aerosol by further compressing the switch. Without further compression of the switch, only the dispensing gas is released from the spray nozzle tip 26. This further dispensing of gas helps in clearing the passages of the nozzle tip 26 in preparation for future use.

As further shown in FIG. 2, the fluid chambers 16, 17 include floating pistons 24 within the cylinders 22. The pistons 24 seal the fluids 14, 15 within the fluid chambers 16, 17 and are movable to compress the fluids 14, 15 into spray nozzle tip 26 upon the application of pressurized gas delivered via the plug outlets 106, 107. Furthermore, the O-ring 112 surrounding the outer portion of the plugs 104, 105 is sealably positioned against the interior of the cylinder 22. Accordingly, pressurized gas within the cylinder 22 may reach a maximum of approximately 20 psi. Therefore, this seal preferably is air tight up to about 20 psi. It will be appreciated that this seal may be designed for various pressures depending on the amount of pressurized gas to be delivered.

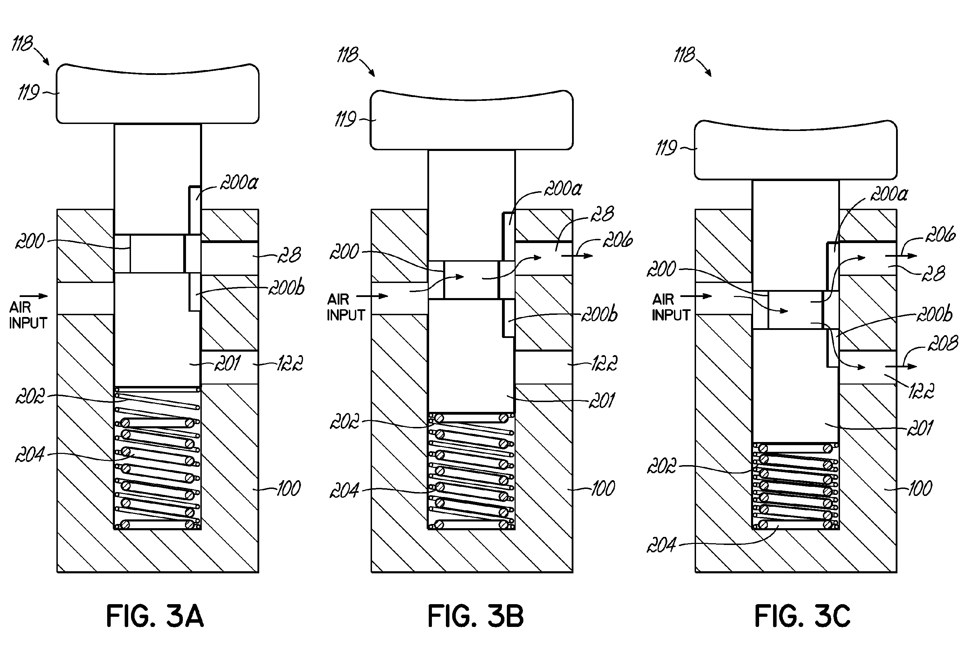

FIGS. 3A-3C show the three-way switch 118 in accordance with one embodiment of the present invention that may be movable through first and second stages. The switch 118 includes an annular groove 200, which, when properly aligned, permits the passage of pressurized gas. It will be appreciated other geometry or through hole by which gas passes may also be used. More particularly, the switch 118 is spring biased by at least a first spring 202 into the normally closed position as shown in FIG. 3A. While closed, the switch 118 operably blocks the pressurized gas from traveling from an input air line to either the sprayer gas line 28 or the material delivery gas line 122. As shown in FIG. 3B a trigger 119 is depressed and such that the switch 118 passes into the first stage which compresses the first spring 202 until a plunger 201 contacts a second spring 204. In this first stage, the switch 118 is compressed or repositioned such that the air input is in fluid communication with the sprayer gas line 28, as indicated by arrows 206. More specifically, the pressurized gas passes through the annular groove 200 and into the sprayer gas line 28. In order to reach the second stage as shown in Fig. 3C, the trigger 119 further compresses the plunger 201 against the second spring 204 until the air input is in fluid communication, via the annular groove 200 and notches 200a, 200b, with both the sprayer gas line 28 and the material delivery gas line 122, as indicated by arrow 208. The first spring 202 may be stiffer than the second spring 204 to differentiate the first stage from the second stage. By doing so, an operator may sense the stages by the differing spring stiffness while compressing the switch 118. Moreover, the three-way switch 118 may be molded or similarly formed into the handle 100. However, it will be appreciated that many switch designs may be used to deliver pressurized gas to these gas lines.

An embodiment of the keyed features is shown in more detail in FIG. 4. To facilitate consistent assembly of the fluid-dispensing device 12 with the holder 30, the first fluid chamber 16 containing the first fluid 14 is loaded into the first channel 32 of the holder 30 while the second fluid chamber 17 containing a second fluid 15 is loaded into the second channel 33 of the holder 30. The distal tapered ends 18 of the fluid chambers 16, 17 each may include one of the two keyed adaptors 220, 222. The first and second keyed adaptors 220, 222 may vary in at least one structural dimension, which, as shown, are different diameters. Each adaptor 220, 222 further includes a distally-extending port, such as a luer, configured to be coupled to the inlets 224, 226 of the nozzle tip 26.

In addition to the keyed adaptors 220, 222, the distal ends of the first and second channels 32, 33 of the holder 30 may be particularly configured to receive one of the two keyed adaptors 220, 222. More specifically and in accordance with the exemplary embodiment, the first channel 32 includes one or more ribs 228 that reduce the inner diameter of the first channel 32 to approximately the outer diameter of the first keyed adaptor 220. Meanwhile, the second channel 33, remaining devoid of diameter-reducing ribs, remains sufficiently large so as to receive the larger outer diameter of the second keyed adaptor 33. As a result, and when the fluid chambers 16 are loaded into the holder 30, the first fluid within the first fluid chamber 16 is necessarily loaded into the first channel 32 of the holder 30 having the ribs 228 therein to support the smaller outer diameter adaptor 220. The second chamber 16 containing the second fluid is then necessarily loaded into the second channel 33.

While the invention has been illustrated by the description of embodiments thereof, and while the embodiments have been described in considerable detail, it is not intended to restrict or in any way limit the scope of the appended claims to such detail. Additional advantages and modifications will readily appear to those skilled in the art. Therefore, the invention in its broadest aspects is not limited to the specific details shown and described. The various features disclosed herein may be used in any combination necessary or desired for a particular application. Consequently, departures may be made from the details described herein without departing from the spirit and scope of the claims which follow. What is claimed is:

## Claims

1. A gas-assisted fluid-dispensing device configured to deliver an aerosol onto a surgical site, the fluid dispensing device comprising:
a support structure;
a fluid chamber coupled with the support structure and configured to contain a fluid therein, the fluid chamber having a distal end and a proximal end;
a piston positioned within the fluid chamber;
a spray nozzle tip fluidly coupled to the distal end of the fluid chamber and having a gas inlet, the spray nozzle tip capable of generating the aerosol with a gas from the gas inlet and the fluid from the fluid chamber; and
a pneumatic switch operably coupled to the support structure and having a first stage configured to permit gas flow through the pneumatic switch and into the gas inlet of the spray nozzle tip and a second stage configured to permit gas flow through the pneumatic switch and into both the gas inlet of the spray nozzle tip and into the fluid chamber for moving the piston and forcing the fluid from the fluid chamber into the spray nozzle tip.

2. The gas-assisted fluid-dispensing device of claim 1, wherein the support structure further comprises a handle, and the fluid-dispensing device further comprises:
a holder configured to support the fluid chamber therein, the holder operably coupled to the handle.

3. The gas-assisted fluid-dispensing device of claim 2 wherein the handle, the holder, and the pneumatic switch are formed as a unitary piece.

4. The gas-assisted fluid-dispensing device of claim 1, wherein the spray nozzle tip, the handle, and the fluid chamber are co-planar.

5. A gas-assisted fluid-dispensing device configured to deliver an aerosol onto a surgical site, the fluid dispensing device comprising:
a support structure;
first and second fluid chambers coupled with the support structure and configured to contain respective first and second fluids therein, the first and second fluid chambers each having a distal end and a proximal end;
first and second pistons positioned respectively within the first and second fluid chambers;
a spray nozzle tip fluidly coupled to the distal ends of the first and second fluid chambers and having a gas inlet, the spray nozzle tip capable of generating the aerosol with a gas from the gas inlet and the first and second fluids from the first and second fluid chambers; and
a pneumatic switch operably coupled to the support structure and having a first stage configured to permit gas flow through the pneumatic switch and into the gas inlet of the spray nozzle tip and a second stage configured to permit gas flow through the pneumatic switch and into both the gas inlet of the spray nozzle tip and into the first and second fluid chambers for moving the respective first and second pistons and forcing the fluids from the first and second fluid chambers into the spray nozzle tip.

6. The gas-assisted fluid-dispensing device of claim 5, wherein the support structure further comprises a handle, and the fluid-dispensing device further comprises:
a holder configured to couple the first and second fluid chambers to the handle, the holder having first and second channels configured to receive respective ones of the first and second fluid chambers; and
first and second keyed adaptors coupled to respective distal ends of the first and second fluid chambers,
wherein the first channel includes a projection configured to receive the keyed adaptor of the first fluid chamber.

7. The gas-assisted fluid-dispensing device of claim 5, further comprising:
a holder configured to couple the first and second fluid chambers to the support structure; and
an arm extending distally away from the holder toward the spray nozzle tip, the arm having a keyed surface,
wherein the spray nozzle tip includes a keyed groove configured to receive the keyed surface of the arm.

8. A gas-assisted fluid-dispensing device configured to deliver an aerosol onto a surgical site, the fluid dispensing device comprising:
a support structure;
a fluid chamber coupled with the support structure and configured to contain a fluid therein, the fluid chamber having a distal end and a proximal end;
a piston positioned within the fluid chamber;
a spray nozzle tip fluidly coupled to the distal end of the fluid chamber and having a gas inlet, the spray nozzle tip capable of generating the aerosol with a gas from the gas inlet and the fluid from the fluid chamber;
a gas inlet operably coupled to the support structure;
a pneumatic switch operably coupled to the support structure;
a first fluid line in fluid communication with the pneumatic switch and the gas inlet operably coupled to the support structure;
a second fluid line in fluid communication with the pneumatic switch and the gas inlet of the spray nozzle tip; and
a third fluid line in fluid communication with the pneumatic switch and the fluid chamber,
wherein the pneumatic switch in a first stage fluidically couples the first fluid line with the second fluid line for supplying gas to the spray nozzle tip and in a second stage fluidically couples the first fluid line with the second and third fluid lines for supplying gas to the spray nozzle tip and to the fluid chamber for moving the piston and forcing the fluid from the fluid chamber into the spray nozzle tip.

9. The gas-assisted fluid-dispensing device of claim 8, further comprising:
a first orifice within the second fluid line; and
a second orifice within the third fluid line,
wherein the first orifice has a diameter that is greater than a diameter of the second orifice.

10. A fluid dispensing device comprising:
a fluid chamber having a distal end, a proximal end, and configured to contain a fluid therein;
a piston positioned within the fluid chamber; and
a plug inserted into the proximal end of the fluid chamber, the plug having a plug outlet; and
a gas inlet operably coupled to the plug outlet to provide pressurized gas into the fluid chamber,
wherein the piston seals the proximal end of the fluid chamber such that the pressurized gas applies pressure to the piston.

11. A method of using a gas-assisted fluid-dispensing device for delivering an aerosol onto a surgical site, the fluid-dispensing device including a fluid chamber containing a fluid therein, a piston positioned within the fluid chamber, a spray nozzle tip fluidly coupled to the fluid chamber and having a gas inlet, and a pneumatic switch, the method comprising:
actuating the pneumatic switch into a first stage to permit gas flow into the gas inlet of the spray nozzle tip;
actuating the pneumatic switch into a second stage to permit gas flow through the pneumatic switch and into both the gas inlet of the spray nozzle tip and into the fluid chamber for moving the piston and forcing the fluid from the fluid chamber into the spray nozzle tip.

12. The method of claim 11, wherein the gas-assisted fluid-dispensing device includes first and second fluid chambers each containing a fluid therein, and first and second pistons positioned respectively within the first and second fluid chambers, and the spray nozzle tip is fluidly coupled to the first and second fluid chambers, and wherein actuating the pneumatic switch into the second stage further comprises:
permitting gas flow through the pneumatic switch and into both of the gas inlet of the spray nozzle tip and into the first and second fluid chambers for moving the first and second pistons and forcing the fluids from the first and second fluid chambers into the spray nozzle tip.
